# EUROPÄISCHE PATENTSCHRIFT

(11) **EP 0 916 644 B1**
(45) Veröffentlichungstag und Bekanntmachung des Hinweises auf die Patenterteilung: **29.08.2001**
(21) Anmeldenummer: 98118093.8
(22) Anmeldetag: 25.09.1998
(51) Int. Cl.: C07C 67/60, C07C 67/39, C07C 69/82

(54) **Hochsiederkreislauf im DMT-Prozess**
Highboiling material recycle in the DMT-process
Recyclage de matières à point d'ébullition élevé dans le procédé de TDM

(30) Priorität: 14.11.1997 DE 19750491
(43) Veröffentlichungstag der Anmeldung: 19.05.1999
(73) Patentinhaber: Degussa AG, 40474 Düsseldorf (DE)
(72) Erfinder: Neutzler, Ulrich, 58300 Wetter (DE); Schoengen, Anton, 58452 Witten (DE)

(56) Entgegenhaltungen:
- EP-A- 0 156 133
- WO-A-90/09367

## Beschreibung

Die Erfindung betrifft ein Verfahren zur Herstellung von rohem Dimethylterephthalat (Roh-DMT) unter Einbeziehung der Rückführung Hochsieder-haltiger Rückstände in den Prozeß, wobei das Verfahren im wesentlichen folgende Verfahrensstufen beinhaltet,
- die Oxidation eines überwiegend para-Xylol (p-X) und para-Toluylsäuremethylester (pT-Ester) enthaltenden Gemisches in flüssiger Phase mit einem Sauerstoff-haltigen Gas in Gegenwart eines Katalysators,
- die Veresterung der in der Oxidation erhaltenen Säuren mit Methanol und
- destillative Auftrennung des aus der Veresterung stammenden Produktgemisches, dem sogenannten Rohester, in a) eine pT-Ester-reiche Fraktion, die in die Oxidation zurückgeführt wird, b) eine Roh-DMT-Fraktion und c) eine schwersiedende Rückstandsfraktion, gegebenenfalls deren Aufarbeitung und Rückgewinnung des Katalysators.

Dimethylterephthalat (DMT) wird großtechnisch in zahlreichen Anlagen weltweit hergestellt. DMT ist eine wichtige Ausgangsverbindung für die Herstellung von Polyestern. Die Anwendungsgebiete der Polyester für Fasern sowie Folien, u. a. für fotographische Filme und Magnetbänder, oder Flaschen aus Polyethylenterephthalat, um nur einige zu nennen, sind lange bekannt.

Es ist bekannt, daß das heutige Witten-DMT-Verfahren im wesentlichen die Verfahrensstufen
- Oxidation von p-X und pT-Ester mit gegebenenfalls nachgeschalteter Abgasreinigung sowie Energierückgewinnung,
- Veresterung der entstandenen organischen Säuren mit Methanol,
- Auftrennung des sogenannten Rohesters in a) eine pT-Ester-reiche Fraktion, die in die Oxidation zurückgeführt wird, b) eine Roh-DMT-Fraktion, mit beispielsweise 90 bis hin zu mehr als 99 Gew.- % DMT und c) eine schwersiedende Rückstandsfraktion, gegebenenfalls deren
- Aufarbeitung, z. B. durch Methanolyse sowie Thermolyse, nachfolgend auch Rückstandsaufarbeitung genannt, und
- Rückgewinnung des meist Kobalt-haltigen Katalysators aus Hochsiederhaltigen Rückstandsfraktionen der Rohesterdestillation bzw. der Rückstandsaufarbeitung,
- und der Reinigung der Roh-DMT-Fraktion, beispielsweise durch Waschen, Umkristallisieren sowie DMT-Reindestillation,
beinhaltet ("Terephthalsäuredimethylester", Ullmann Bd. 22, 4. Auflage, S. 529 - 533; EP 0 464 046 B1; DE-OS 40 26 733). Es ist ferner bekannt, aus DMT, DMT-reinst oder auch aus DMT-reichen Fraktionen durch eine gezielte Hydrolyse Terephthalsäure (TA) in unterschiedlicher Reinheit (PTA, PTA-p) herzustellen (vgl. u. a. die DE-Patentanmeldung Nr. 195 36 814.2).

Ein wichtiger Wirtschaftlichkeitsfaktor bei der Herstellung von DMT ist eine möglichst quantitative Nutzung der Einsatzstoffe.

Die Oxidation eines Gemisches aus para-Xylol (p-X) und para-Toluylsäuremethylester (p-TE oder pT-Ester) wird im allgemeinen mit Luftsauerstoff in Gegenwart eines Schwermetall-haltigen Oxidationskatalysators (DE-PS 20 10 137), nachfolgend auch kurz Katalysator genannt, bei einer Temperatur von etwa 140 bis 180 °C und einem Druck von etwa 4 bis 10 bar abs. in flüssiger Phase durchgeführt. Aus der Oxidationsstufe resultiert ein Reaktionsgemisch, nachfolgend auch Oxidat genannt, das überwiegend Monomethylterephthalat (MMT), p-Toluylsäure (p-TA) und Terephthalsäure (TA), gelöst bzw. suspendiert in pT-Ester, enthält. Das Oxidat wird vor Eintritt in die Veresterung üblicherweise in einem sogenannten Oxidataufheizer erhitzt und in der Veresterung mit Methanol bei einer Temperatur von etwa 230 bis 280 °C und einem Druck von 20 bis 25 bar abs. verestert. Der erhaltene Rohester wird in der Rohesterdestillation in eine pT-Ester-reiche Fraktion, eine Roh-DMT-Fraktion und eine hochsiedende, katalysatorhaltige Rückstandsfraktion aufgetrennt.

In den besagten Verfahrensstufen fallen neben den erwünschten Wertprodukten auch Nebenprodukte an. Nachfolgend werden die hochsiedenden, organischen Anteile schwersiedender bzw. hochsiedender Rückstände auch als Hochsieder ("high Boiler" = HB) bezeichnet. Die niedrigsiedenden Anteile einer Rückstandsfraktion werden "Low Boiler" (LB) genannt.

Die aus der Rohesterdestillation hervorgehende pT-Ester-reiche Fraktion wird in die Oxidation zurückgeführt und die Roh-DMT-Fraktion über nachfolgende Reinigungsstufen in an sich bekannter Weise in die gewünschten DMT- oder TA-Produktqualitäten überführt.

Die aus der Rohesterdestillation stammende hochsiedende, katalysatorhaltige Rückstandsfraktion wird im allgemeinen einer Thermolyse oder einer Methanolyse, wie es u. a. der deutschen Patentanmeldung Nr. 195 30 970 und der noch nicht veröffentlichten deutschen Patentanmeldung Nr. 197 24 390.8 zu entnehmen ist, sowie einer Katalysatorrückgewinnung unterzogen.

Aus der DE-PS 29 23 681 geht ein Verfahren zur Gewinnung und Wiederverwertung von Schwermetalloxidationskatalysator aus dem Witten-DMT-Prozeß hervor, wobei der Katalysator durch Extraktion besagter hochsiedender, katalysatorhaltiger Rückstände wiedergewonnen und eine so erhaltene katalysatorhaltige Extraktionslösung, die auch Trimellitsäure (TMS) und Trimellitsäuremethylester (TMME) enthält, in die Oxidation zurückführt wird. Bei der Katalysatorrückgewinnung verbleibt ein hochsiedender, im wesentlichen katalysatorfreier Rückstand.

Die DE-OS 29 23 681 lehrt darüber hinaus, daß eine zu hohe Katalysatorkonzentration zu Verstopfung durch Abscheidung von Katalysatormetall in der Veresterung führt und man die Verstopfung durch möglichst geringe Katalysatormengen in der Oxidation vermeiden kann. Jedoch geht mit abnehmender Katalysatorkonzentration in der Oxidation die Produktausbeute und damit auch die Wirtschaftlichkeit des Verfahrens zurück.

Auch die DE-OS 32 44 915 offenbart ein Verfahren zur Gewinnung von Kobaltlösung und ihrer Verwendung als Katalysatorlösung.

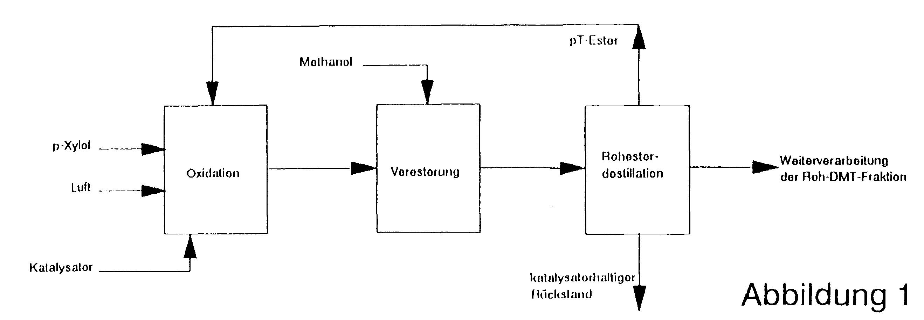

In den Abbildungen 1 bis 4 sind Blockdiagramme an sich bekannter Anordnungen von Teilanlagen einschließlich entsprechender Stoffströme zur Herstellung von Roh-DMT nach dem Witten-DMT-Verfahren dargestellt.

Abbildung 1 zeigt eine weniger zeitgemäße Anordnung der Verfahrensstufen Oxidation, Veresterung und Rohesterdestillation, wobei der katalysatorhaltige, hochsiedende Rückstand aus der Rohesterdestillation vollständig ausgeschleust und beispielsweise einer Verbrennung zugeführt wird. Kobalt ist dann den Verbrennungsabgasen als Oxidstaub zu entnehmen, wodurch in den Rückstandsverbrennungen erhebliche Komplikationen durch Staubablagerungen und erhöhte Aufwendungen für Umweltschutzmaßnahmen entstehen.

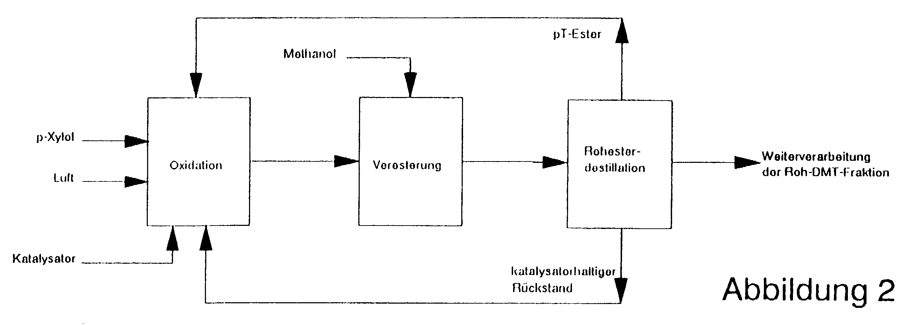

Der Abbildung 2 ist eine der Abbildung 1 vergleichbare Anlagenschaltung zu entnehmen, jedoch ist hier im wesentlichen eine Rückführung des katalysatorhaltigen, hochsiedenden Rückstands aus der Rohesterdestillation in die Oxidation vorgesehen. Die Kreislaufführung von katalysatorhaltigem Rückstand aus der Rohesterdestillation bewirkt jedoch eine nicht unerhebliche Temperaturerhöhung in der Oxidation und damit Ausbeutenachteile bei der Oxidation der beiden Hauptkomponenten p-Xylol und pT-Ester.

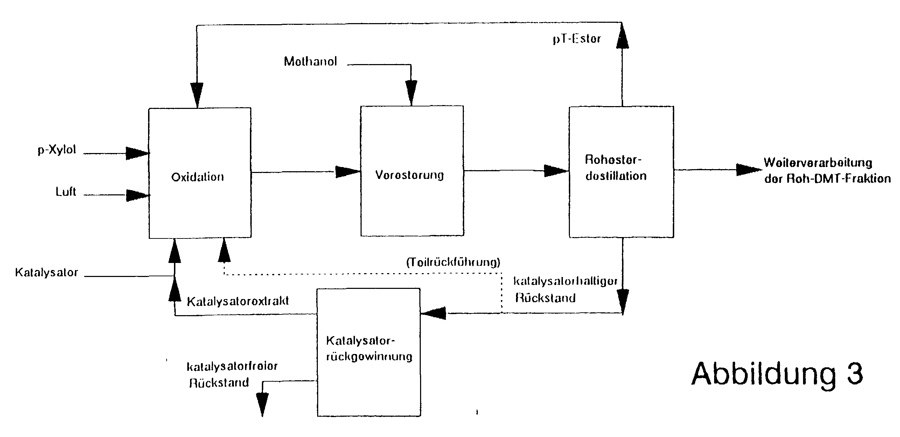

Aus Abbildung 3 geht eine Anlagenvariante hervor, wobei einerseits nur eine Teilrückführung des Rückstands aus der Rohesterdestillation in die Oxidation praktiziert und andererseits der Katalysator aus dem Rückstand der Rohesterdestillation wiedergewonnen und wie oben bereits beschrieben in Form einer Extraktionslösung, gegebenenfalls nach Einengung, in die Oxidation zurückgeführt wird. Der in der Katalysatorückgewinnung anfallende, nunmehr im wesentlichen katalysatorfreie, hochsiedende Prozeßrückstand wird üblicherweise ausgeschleust.

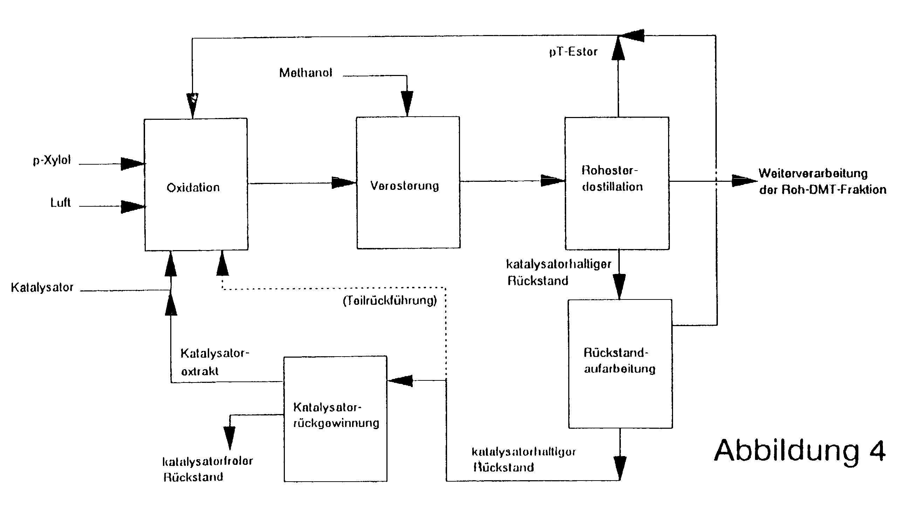

Abbildung 4 gibt das Blockschema eines ebenfalls neueren Anlagentyps wieder. Gegenüber Abbildung 3 ist eine solche Anlage zusätzlich mit einer Thermolyse- oder einer Methanolyse-Stufe zur Aufarbeitung des hochsiedenden Rückstands aus der Rohesterdestillation - nachfolgend Rückstandsaufarbeitung genannt - ausgestattet.

In einer Thermolyse wird der Rückstand aus der Rohesterdestillation bei erhöhter Temperatur einer umesterungsähnlichen Reaktion unterworfen. Die sich bildenden leichtsiedenden Ester werden unter Vakuum abgestrippt und als Wertprodukte in den Prozeß zurückgeführt.

In einer Methanolyse werden besagte Rückstände unter Zusatz von Methanol bei Temperaturen um 250 °C für die Wiederverwertung im Prozeß aufgeschlossen. Solche Anlagen arbeiten mit sehr hohen Ausbeuten und sind solchen ohne Methanolyse aus wirtschaftlicher Sicht im allgemeinen überlegen.

Auch bei solchen Verfahrensvarianten gemäß Abbildung 4 wird üblicherweise nur der wiedergewonnene Katalysator in Form seiner Extraktionslösung in die Oxidation zurückgeführt sowie gegebenenfalls eine Teilrückführung des katalysatorhaltigen, hochsiedenden Rückstands aus der Rückstandsaufarbeitung in die Oxidation vorgesehen.

Darüber hinaus ist bekannt, daß aufgrund schwieriger Prozeßbedingungeninsbesondere vor dem Hintergrund der geringen Löslichkeit sowie der hohen Schmelzpunkte der jeweils auftretenden Reaktionsprodukte - häufig Ablagerungen und Verstopfung in den Anlagenteilen, die insbesondere mit Katalysator in Berührung kommen, auftreten.

Besonders anfällig sind die mit dem Oxidat in Berührung kommenden Apparate und Rohrleitungen. Während es in der Oxidation unter Einhaltung bestimmter maximaler Säurezahlen gelingt, mit entsprechenden Spülungen im wesentlichen ungestörte Betriebsintervalle zu erreichen, kommt es im Oxidataufheizer, in der Veresterung, der Rohesterdestillation, der Rückstandsaufarbeitung, den verbindenden Rohrleitungen und der Katalysatorrückgewinnung immer wieder zu erheblichen Betriebsstörungen durch Verstopfung und Ablagerungen. Während sich in den Leitungen zur Veresterung und dem Oxidataufheizer vor der Veresterung vor allen Dingen Kobaltverbindungen und Terephthalsäure ablagern, handelt es sich in den Veresterungsreaktoren selbst um magnetische bzw. metallische Kobaltablagerungen, die nur durch Befahren der Anlagenteile und manueller Reinigung zu beheben sind. Aus Kobaltmetall gebildete Ablagerungen können so zur völligen Blockierung beispielsweise eines Bodens der Reaktionskolonne und damit zur vollständigen Unterbrechung der Produktion führen.

Während es beim Oxidataufheizer gelingt, durch kurze Spülzyklen mit pT-Ester die Teile für vergleichsweise kurze Zeitintervalle freizuspülen, sind für die Veresterungskolonne und die daran anschließenden Rohrleitungen und besagte Anlagenteile keine geeigneten Verfahren bekannt.

Neben den metallischen Ablagerungen wurden weitere Ablagerungen beobachtet, die praktisch nur aus kristallinem Kobalterephthalat bestehen. Solche Ablagerungen bewirken neben ernsten Betriebsstörungen auch, daß erhebliche Mengen des als Oxidationskatalysator benutzten Kobalts in der Anlage verbleiben, Kobalt nicht in Form von Extrakt wiedergewonnen und deshalb nicht in den Prozeß zurückgeführt werden kann, sondern in adäquater Menge als Frischkatalysator eingesetzt werden muß. Die abgelagerten Kobaltverbindungen im allgemeinen und die magnetischen bzw. metallischen Verbindungen im besonderen lassen sich nur schwierig bzw. verbunden mit sehr hohen Kosten wieder zu geeignetem Katalysator aufarbeiten.

Die Beeinträchtigung der Anlagenverfügbarkeit durch verstopfungsbedingte Störungen bis hin zur Katalysatorrückgewinnung blieben trotz aller bisherigen Bemühungen erhalten, so daß die Vorteile vorliegender Anlagenvarianten bisher nur eingeschränkt nutzbar waren.

Der Erfindung lag somit die Aufgabe zugrunde, im Rahmen des Witten-DMT-Verfahrens Maßnahmen bereitzustellen, die es ermöglichen, die Neigung zu Verstopfung in Anlagenteilen, die nach der Oxidation mit Katalysator in Berührung kommen, zu verringern, um die Wirtschaftlichkeit des Prozesses weiter zu verbessern.

Die gestellte Aufgabe wird erfindungsgemäß entsprechend den Angaben der Patentansprüche gelöst.

Überraschender Weise wurde nun gefunden, daß man durch eine vollständige oder teilweise Rückführung bzw. Einspeisung eines methanolisierten, d. h. in einer vorangehenden Methanolysestufe behandelten, hochsiedenden und im wesentlichen katalysatorfreien Rückstands aus der Katalysatorrückgewinnung in die Oxidation, in die Veresterung, in die Rohesterdestillation, gegebenenfalls in die Rückstandsaufarbeitung sowie in die Katalysatorrückgewinnung und/oder in einen oder in mehrere Stoffströme zwischen den zuvor genannten Verfahrensstufen die Neigung zu Verstopfung in besagten Anlagenteilen, die nach der Oxidation mit Katalysator in Berührung kommen, insbesondere im Bereich zwischen Oxidation und Versterung, in der Veresterung sowie den nachgeschalteten Rohrleitungen und nachfolgenden Anlagenteilen bis hin zur Katalysatorrückgewinnung, deutlich verringert und man daher auch die Zeitabstände zwischen den Reinigungsmaßnahmen in den Anlagen verlängern kann und ferner außerplanmäßige, durch Verstopfung bedingte Abstellungen praktisch entfallen. Darüber hinaus wurde gefunden, daß die Maßnahme, methanolisierten, hochsiedenden, im wesentlichen katalysatorfreien Prozeßrückstand aus der Katalysatorrückgewinnung erfindungsgemäß zurückzuführen, vorzugsweise bei Einspeisung nach der Oxidation bis hin in die Veresterung, die Wirtschaftlichkeit des gesamten Prozesses in hervorragender Weise verbessert.

Gegenstand der vorliegenden Erfindung ist daher ein Verfahren zur Herstellung von rohem Dimethylterephthalat (Roh-DMT) unter Einbeziehung der Rückführung Hochsieder-haltiger Rückstände in den Prozeß, wobei das Verfahren im wesentlichen folgende Verfahrensstufen beinhaltet,
- Oxidation eines überwiegend para-Xylol (p-X) und para-Toluylsäuremethylester (pT-Ester) enthaltenden Gemisches in flüssiger Phase mit einem Sauerstoff-haltigen Gas in Gegenwart eines Katalysators,
- Veresterung der in der Oxidation erhaltenen Säuren mit Methanol,
- destillative Auftrennung des aus der Veresterung stammenden Produktgemisches, dem sogenannten Rohester, in
   a) eine pT-Ester-reiche Fraktion, die in die Oxidation zurückgeführt wird,
   b) eine Roh-DMT-Fraktion und
   c) eine hochsiedende Rückstandsfraktion, gegebenenfalls deren Aufarbeitung, und Rückgewinnung des Katalysators,
das dadurch gekennzeichnet ist, daß man die hochsiedende Rückstandsfraktion aus der Rohesterdestillation, bevor sie der Katalysatorrückgewinnung zugeführt wird, über eine Rückstandsaufarbeitung fährt, die (i) eine Methanolyse oder (ii) eine Thermolyse und eine nachgeschaltete Methanolyse beinhaltet, und den Rückstand aus der Katalysatorrückgewinnung durch eine vollständige oder teilweise Einspeisung desselben in die Oxidation, in die Veresterung, in die Rohesterdestillation, gegebenenfalls in die Rückstandsaufarbeitung, in die Katalysatorrückgewinnung und/oder in einen oder in mehrere Stoffströme zwischen den zuvor genannten Verfahrensstufen in den Prozeß zurückführt.

Es ist besonders überraschend, daß eine so einfache Maßnahme zur Lösung der Aufgabe führt und somit den Prozeß in einfacher Weise, bei hohen Ausbeuten und ohne Beeinträchtigung der Laufzeit der Anlage, die durch Ablagerungen und Feststoffbildung bedingt sind, zu betreiben.

Im allgemeinen führt man das vorliegende Verfahren im Rahmen des Witten-DMT-Verfahrens derart aus, daß man einen methanolisierten, im wesentlichen katalysatorfreien, hochsiedenden Rückstand aus der Katalysatorrückgewinnung vollständig oder teilweise in die Oxidation, in die Veresterung, in die Rohesterdestillation, gegebenenfalls in die Rückstandsaufarbeitung, in die Katalysatorrückgewinnung und/oder in einen oder in mehrere Stoffströme zwischen den zuvor genannten Verfahrensstufen einspeist und somit nutzbringend in den Prozeß zurückführt.

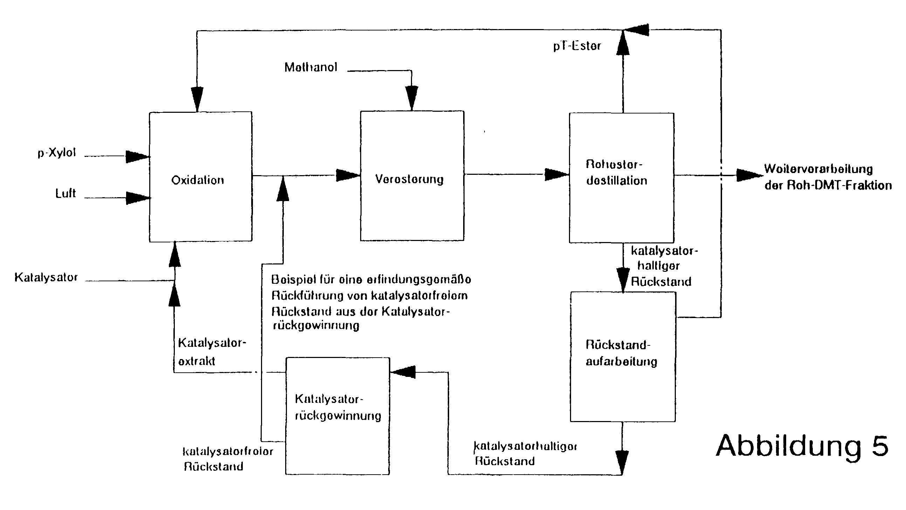

Abbildung 5 zeigt als Beispiel das Blockschema einer bevorzugten Ausführungform des erfindungsgemäßen Verfahrens.

Vorzugsweise führt man das erfindungsgemäße Verfahren derart aus, daß man einen methanolisierten, im wesentlichen katalysatorfreien Rückstand aus der Katalysatorrückgewinnung vollständig oder teilweise in den Stoffstrom zwischen Oxidation und Veresterung und/oder in die Veresterung fährt.

Beim erfindungsgemäßen Verfahren wird der Rückstand aus der Katalysatorrückgewinnung geeigneterweise in Menge von > 0 bis 20 Gew.-%, vorzugsweise 1 bis 20 Gew.-%, besonders vorzugsweise 2 bis 20 Gew.-%, ganz besonders vorzugsweise 6 bis 12 Gew.-%, bezogen auf die jeweils aktuell produzierte Roh-DMT-Menge zurückgeführt; es können aber auch größere Mengen als 20 Gew.-% zurückgeführt werden. Die Katalysatorkonzentration in der Oxidation beträgt beim erfindungsgemäßen Verfahren geeigneterweise 100 bis 200 Gew.-ppm, insbesondere 130 bis 170 Gew.-ppm.

Beispielsweise können bei 2 Gew.-% erfindungsgemäßer Rückstandsrückführung u. a. nachfolgend aufgeführte vorteilhafte Auswirkungen festgestellt werden:
- Die Spülung des Oxidataufheizers mit pT-Ester kann unterbleiben.
- Feststoffbildungen während der Methanolyse waren nicht feststellbar.
- Die Veresterung in einer erfindungsgemäß betriebenen Anlage war nach 3/4-jährigem Betrieb metallisch blank, ein vorher nie beobachteter Zustand.
- Die TA-Abscheidung bei der Extraktion ging um mehr als 50 % zurück und bereitete entsprechend weniger Probleme.

Vorzugsweise fährt man beim erfindungsgemäßen Verfahren die hochsiedende Rückstandsfraktion aus der Rohesterdestillation, bevor sie der Katalysatorrückgewinnung zugeführt wird, über eine Rückstandsaufarbeitung, die (i) eine Methanolyse oder (ii) eine Thermolyse und eine nachgeschaltete Methanolyse beinhaltet.

Somit wird der Rückstand aus der Rohesterdestillation beim erfindungsgemäßen Verfahren vor Eintritt in die Katalysatorrückgewinnung vorteilhafterweise methanolytisch behandelt. Die Behandlung in der Rückstandsaufarbeitung sollte geeigneterweise bei einer Temperatur erfolgen, die nicht oberhalb von 260 °C liegt.

Eine Verfahrensvariante im Rahmen des vorliegenden DMT-Prozesses sieht u. a. vor, die Roh-DMT-Fraktion vor der Zuführung in die Kristallisation einer erneuten fraktionierten Destillation, der sogenannten DMP-Destillation, zu unterziehen. Hierbei fällt ein Rückstand an, der im wesentlichen die in der Roh-DMT-Fraktion enthaltenen Schwersieder bzw. Hochsieder enthält.

Geeigneterweise kann man beim erfindungsgemäßen Verfahren der schwersiedenden Rückstandsfraktion aus der Rohesterdestillation vor Eintritt in die Methanolyse den Rückstand aus der DMP-Destillation ganz oder teilweise hinzufügen und den Rest des Rückstands aus der DMP-Destillation in die Oxidation fahren.

Beim erfindungsgemäßen Verfahren ist ganz besonders eine Methanolyse geeignet, die mit einer Mehrfacheinleitung von Methanol betrieben wird, so wie es aus der noch nicht veröffentlichten deutschen Patentanmeldung Nr. 197 24 390.8 hervorgeht.

Auch ist es bevorzugt, wenn man beim erfindungsgemäßen Verfahren die hochsiedende Rückstandsfraktion aus der Rohesterdestillation vor Eintritt in die Methanolyse über eine Nachdestillation und/oder eine Methanolysevorstufe fährt. Vorteilhafterweise kann man zusätzlich das Destillat der Nachdestillation über eine sogenannte TMT-Destillation fahren und/oder teilweise in die Rohesterdestillation zurückführen.

Vorzugsweise wird beim erfindungsgemäßen Verfahren der Rücklauf einer TMT-Destillation so eingestellt, daß das TMT im Sumpf verbleibt. Das Sumpfprodukt der TMT-Destillation kann in solchen Mengen mit dem Rückstand aus der Katalysatorrückgewinnung erfindungsgemäß vor und/oder in die Veresterung zurückgeführt werden, so daß der TMT-Spiegel im Rohester 0,5 bis 1 Gew.-% nicht übersteigt.

Beim erfindungsgemäßen Verfahren ist es ferner von Vorteil, wenn man vorzugsweise den in der Methanolyse entstehenden Brüden in eine leicht- und eine schwersiedende Phase zerlegt, den im Brüden enthaltenen Anteil an Trimellitsäuretrimethylester (TMT) dem Sumpfprodukt der Methanolyse zuführt, anteilig das mit TMT angereicherte Sumpfprodukt ausschleust oder der schwersiedenden Rückstandsfraktion aus der Katalysatoraufarbeitung hinzufügt.

Das erfindungsgemäße Verfahren erlaubt es darüber hinaus in hervorragender Weise als Rückstandsaufarbeitung insbesondere eine Methanolyse und eine Katalysatorrückgewinnung ohne Beeinträchtigung durch Feststoffbildung zu betreiben und den Prozeß bevorzugt nur mit extrahiertem Katalysator zu fahren, ohne daß sich das Co/Mn-Verhältnis des Katalysators nennenswert verschiebt. Dem Prozeß kann jedoch auch Frischkatalysator zugeführt werden.

Ein weiterer Vorteil beim erfindungsgemäßen Verfahren besteht darin, daß man auf lonentauscheranlagen für die Einstellung des TMS-Spiegels bei der Katalysatorrückgewinnung verzichten kann und darüber hinaus auch die Konzentration der dünnen Extraktlösung ohne Beeinträchtigung des Betriebes in kontinuierlichen Eindampfanlagen bis auf eine Konzentration von rund 30 g/l Co oder mehr vornehmen kann.

Ferner können Anlagen gemäß Abbildung 1 bis 4 unter erheblicher Ausbeuteverbesserung für eine erfindungsgemäße Betriebsweise umgebaut werden. Dies gilt insbesondere dann, wenn in geeigneter Weise eine Methanolyse vorgesehen wird.

Im allgemeinen enthalten die hochsiedenden Rückstände, die erfindungsgemäß rückgeführt werden, Stoffe, wie z. B. Diphenyle und Terphenyle mit einer oder mehreren Methylester- und/oder Säuregruppen, wovon ein Teil ortho-substituiert ist sowie 10 bis 50 % trimellitische Substanzen. Beim erfindungsgemäßen Verfahren wird die Trimellitsäure in einer Methanolyse in der Regel gleich hier verestert und destillativ abgetrennt, wobei die di- und terphenylischen Ester vorzugsweise im Rückstand verbleiben. Vorteilhafterweise sollte bei einer solchen Fahrweise die Methanolmenge so eingestellt werden, daß der DMT-Gehalt der Hochsieder abstrippt und die DMT-Konzentration vorzugsweise 0,5 bis 1 % im methanolisierten Rückstand nicht übersteigt.

Es ist bekannt, daß die in herkömmlichen Anlagen mit einer Methanolyse in der Katalysatorrückgewinnung anfallenden Rückstände auch erhebliche Mengen an TPA und MMT enthalten. In Anlagen, die erfindungsgemäß betrieben werden, sind diese Stoffe im hochsiedenden Rückstand aus der Katalysatorrückgewinnung in der Regel nur in so geringen Mengen vorhanden, daß sie löslich bleiben und auch keine Feststoffprobleme mehr auftreten.

Außerdem wurde gefunden, daß TMS, die in bisherigen Anlagen mit Rückstandskreisläufen insbesondere im Katalysatorextrakt beobachtet wurde, bei erfindungsgemäßem Betreiben, an besagter Stelle vorteilhafterweise nicht mehr auftritt.

Somit kann der katalysatorhaltige Extrakt aus der Katalysatoraufarbeitung auch ohne eine zusätzliche Reinigung, z. B. mittels lonentauscher zur Entfernung von TMS, direkt in die Oxidation gefahren werden, so daß eine inhibierende Wirkung auf das Katalysatorsystem in der Oxidation, die von TMS ausgehen kann, in vorteilhafter Weise unterbleibt. Aus gleichen Gründen wirkt sich das erfindungsgemäße Verfahren auch dadurch vorteilhaft aus, daß es nicht mehr erforderlich wird, einen inhibitorhaltigen Rückstand, wie z. B. einen nicht methanolisierten Rückstand aus der Rohesterdestillation, vgl. die Abbildungen 2 und 3, in die Oxidation zurückzuführen.

Durch das erfindungsgemäße Verfahren kann die Wirtschaftlichkeit sowohl in älteren als auch in neueren Anlagen, wie sie den Abbildungen 1 bis 4, aber auch der DE-PS 29 16 197 und der EP 0 464 046 B1 zu entnehmen sind, entscheidend verbessert werden.

### Legende zu Abbildung 1:

Vereinfachtes Blockschema für ein älteres Anlagenschema zur Herstellung von Roh-DMT - hier: mit Oxidation, Veresterung und Rohesterdestillation, wobei der hochsiedende, katalysatorhaltige Rückstand aus der Rohesterdestillation ausgeschleust wird.

### Legende zu Abbildung 2:

Beispiel für ein Anlagenschema zur Herstellung von Roh-DMT - hier: mit Oxidation, Veresterung und Rohesterdestillation, wobei der hochsiedende, katalysatorhaltige Rückstand aus der Rohesterdestillation im wesentlichen in die Oxidation zurückgeführt wird.

### Legende zu Abbildung 3:

Beispiel für einen neueren Anlagentyp zur Herstellung von Roh-DMT - hier: mit Oxidation, Veresterung, Rohesterdestillation und Katalysatorrückgewinnung, wobei das Katalysatorextrakt sowie der hochsiedende, katalysatorhaltige Rückstand aus der Rohesterdestillation anteilig in die Oxidation geführt werden.

### Legende zu Abbildung 4:

Blockschema für einen neueren Anlagentyp zur Herstellung von Roh-DMT-dieser besitzt die gleiche Ausstattung wie der in Abbildung 3 und verfügt darüber hinaus über eine Rückstandsaufarbeitung, wobei die Leichtsieder aus der Rückstandsaufarbeitung dem pT-Ester-Strom zugeführt werden.

### Legende zu Abbildung 5:

Abbildung 5 zeigt ein vereinfachtes Blockschema einer bevorzugten Ausfühungsform des erfindungsgemäßen Verfahrens ohne den Gegenstand der vorliegenden Erfindung zu beschränken.

### Legende der Abkürzungen:

- p-X :: para-Xylol
- p-TA :: para-Toluylsäure
- p-TE :: para-Toluylsäuremethylester (pT-Ester)
- BME :: Benzoesäuremethylester
- HM-BME :: Hydroxymethylbenzoesäuremethylester
- MM-BME :: Methoxymethylbenzoesäuremethylester
- DMT :: Dimethylterephthalat
- DMT-roh = RE :: Rohester (DMT-Rohesterstrom nach der Veresterung)
- Roh-DMT :: Dimethylterephthalatfraktion nach der Rohester-Destillation
- DMT-reinst :: Dimethylterephthalat-reinst (hochreines DMT)
- DMP :: Dimethylphthalate = Isomerengemisch aus DMT, Dimethylorthopthalsäure (DMO), Dimethlisophthalsäure (DMI)
- MMT :: Monomethylterephthalat (Terephthalsäuremonomethylester)
- TA :: Terephthalsäure
- MTA :: mittelreine Terephthalsäure
- PTA :: Terephthalsäure hoher Reinheit
- PTA-p :: Terephthalsäure sehr hoher, d. h. höchster Reinheit (Gehalt an MMT und p-TA von zusammen < 50 Gew.-ppm)
- TAS :: Terephthalaldehydsäure (4-CBA)
- TAE :: Terephthalaldehydsäuremethylester
- TMT :: Trimellitsäuretrimethylester
- TMME :: Trimellitsäuremonomethylester
- TMS :: Trimellitsäure
- HB :: hochsiedender Anteil im Rückstand = "high boilern
- LB :: niedrigsiedende Produkte im Rückstand = "low boiler"

## Patentansprüche

1. Verfahren zur Herstellung von rohem Dimethylterephthalat (Roh-DMT) unter Einbeziehung der Rückführung Hochsieder-haltiger Rückstände in den Prozeß, wobei das Verfahren im wesentlichen folgende Verfahrensstufen beinhaltet,
- Oxidation eines überwiegend para-Xylol (p-X) und para-Toluylsäuremethylester (pT-Ester) enthaltenden Gemisches in flüssiger Phase mit einem Sauerstoff-haltigen Gas in Gegenwart eines Katalysators,
- Veresterung der in der Oxidation erhaltenen Säuren mit Methanol,
- destillative Auftrennung des aus der Veresterung stammenden Produktgemisches, dem sogenannten Rohester, in
a) eine pT-Ester-reiche Fraktion, die in die Oxidation zurückgeführt wird,
b) eine Roh-DMT-Fraktion und
c) eine hochsiedende Rückstandsfraktion, deren Aufarbeitung, und Rückgewinnung des Katalysators,
**dadurch gekennzeichnet**,
daß man die hochsiedende Rückstandsfraktion aus der Rohesterdestillation, bevor sie der Katalysatorrückgewinnung zugeführt wird, über eine Rückstandsaufarbeitung fährt, die (i) eine Methanolyse oder (ii) eine Thermolyse und eine nachgeschaltete Methanolyse beinhaltet, und den Rückstand aus der Katalysatorrückgewinnung durch eine vollständige oder teilweise Einspeisung desselben in die Oxidation, in die Veresterung, in die Rohesterdestillation, gegebenenfalls in die Rückstandsaufarbeitung, in die Katalysatorrückgewinnung und/oder in einen oder in mehrere Stoffströme zwischen den zuvor genannten Verfahrensstufen in den Prozeß zurückführt.

2. Verfahren nach Anspruch 1,
**dadurch gekennzeichnet**,
daß man den in der Methanolyse entstehenden Brüden in eine leicht- und eine schwersiedende Phase zerlegt, den im Brüden enthaltenen Anteil an Trimellitsäuretrimethylester (TMT) dem Sumpfprodukt der Methanolyse zuführt, anteilig das mit TMT angereicherte Sumpfprodukt ausschleust oder der schwersiedenden Rückstandsfraktion aus der Katalysatoraufarbeitung hinzufügt.

3. Verfahren nach Anspruch 1 oder 2,
**dadurch gekennzeichnet**,
daß man der schwersiedenden Rückstandsfraktion aus der Rohesterdestillation vor Eintritt in die Methanolyse den Rückstand aus der DMP-Destillation ganz oder teilweise hinzufügt und den Rest des Rückstands aus der DMP-Destillation in die Oxidation führt.

4. Verfahren nach mindestens einem der Ansprüche 1 bis 3,
**dadurch gekennzeichnet**,
daß man die schwersiedende Rückstandsfraktion aus der Rohesterdestillation über eine Methanolyse fährt, die mit einer Mehrfacheinleitung von Methanol betrieben wird.

5. Verfahren nach mindestens einem der Ansprüche 1 bis 4,
**dadurch gekennzeichnet**,
daß man die schwersiedende Rückstandsfraktion aus der Rohesterdestillation vor Eintritt in die Methanolyse über eine Nachdestillation und/oder eine Methanolysevorstufe fährt.

6. Verfahren nach Anspruch 5,
**dadurch gekennzeichnet**,
daß man das Destillat der Nachdestillation über eine TMT-Destillation fährt und/oder teilweise in die Rohesterdestillation zurückführt.

7. Verfahren nach mindestens einem der Ansprüche 1 bis 6,
**dadurch gekennzeichnet**,
daß die Menge der zurückgeführten, hochsiedenden Rückstände aus der Katalysatorrückgewinnung > 0 bis 20 Gew.-% der im laufenden Prozeß hergestellten Roh-DMT-Menge beträgt.

## Claims

1. A process for preparing crude dimethyl terephthalate (crude-DMT) incorporating the recycling of high-boiler-containing residues to the process, the process essentially including the following process stages,
- oxidation of a mixture predominantly comprising para-xylene (p-X) and para-toluic acid methyl ester (pT-ester) in the liquid phase with an oxygen-containing gas in the presence of a catalyst,
- esterification with methanol of the acids present in the oxidation,
- fractionation by distillation of the product mixture originating from the esterification, the so-called crude ester, into
a) a pT-ester-rich fraction which is. recirculated to the oxidation,
b) a crude-DMT fraction and
c) a high-boiling residue fraction, its work-up, and recovery of the catalyst,
**characterized in that** the high-boiling residue fraction is run from the crude ester distillation, before it is fed to the catalyst recovery, through a residue work-up which includes (i) a methanolysis or (ii) a thermolysis and a downstream methanolysis, and the residue from the catalyst recovery is recirculated to the process by complete or partial feed of the same to the oxidation, to the esterification, to the crude ester distillation, if desired to the residue work-up, to the catalyst recovery and/or to one or more mass streams between the abovementioned process stages.

2. A process according to claim 1, **characterized in that** the vapour produced in the methanolysis is fractionated into a low-boiling phase and a high-boiling phase, the content of trimellitic acid trimethyl ester (TMT) present in the vapour is fed to the bottom product of the methanolysis, and the TMT-enriched bottom product is proportionately ejected or added to the high-boiling residue fraction from the catalyst work-up.

3. A process according to claim 1 or 2, **characterized in that** the residue from the DMP distillation is added, in whole or in part, to the high-boiling residue fraction from the crude ester distillation prior to entry into the methanolysis, and the remainder of the residue from the DMP distillation is run into the oxidation.

4. A process according to at least one of claims 1 to 3, **characterized in that** the high-boiling residue fraction from the crude ester distillation is run through a methanolysis which is operated with a multiple introduction of methanol.

5. A process according to at least one of claims 1 to 4, **characterized in that** the high-boiling residue fraction from the crude ester distillation is, prior to entry into the methanolysis, run through a secondary distillation and/or a methanolysis preliminary stage.

6. A process according to claim 5, **characterized in that** the distillate of the secondary distillation is run through a TMT distillation and/or is partially recirculated to the crude ester distillation.

7. A process according to at least one of claims 1 to 6, **characterized in that** the amount of the recirculated high-boiling residues from the catalyst recovery is > 0 to 20% by weight of the crude-DMT amount produced in the running process.

## Revendications

1. Procédé de préparation de téréphtalate de diméthyle brut (DMT brut) impliquant le recyclage de résidus contenant des substances à point d'ébullition élevé dans le processus, le procédé incluant essentiellement les étapes opératoires suivantes :
- oxydation d'un mélange contenant d'une manière prépondérante du paraxylène (p-X) et de l'ester méthylique d'acide toluylique (ester pT) en phase liquide, avec un gaz contenant de l'oxygène en présence d'un catalyseur,
- estérification des acides obtenus dans l'oxydation avec du méthanol,
- séparation par distillation du mélange de produits provenant de l'estérification, ce que l'on appelle l'ester brut, en
a) une fraction riche en ester pT qui est ramenée dans l'oxydation,
b) une fraction de DMT brut et
c) une fraction de résidu à haut point d'ébullition, sa purification et la récupération du catalyseur,
**caractérisé en ce qu**'
on fait passer la fraction de résidu à haut point d'ébullition provenant de la distillation de l'ester brut, avant qu'elle soit amenée à la récupération du catalyseur, par une purification du résidu qui inclut i) une méthanolyse ou ii) une thermolyse, et une méthanolyse post-connectée,
et on ramène le résidu provenant de la récupération du catalyseur par une alimentation complète ou partielle de celui-ci dans l'oxydation, dans l'estérification, dans la distillation de l'ester brut, éventuellement dans la purification du résidu, dans la récupération du catalyseur et/ou en un ou en plusieurs courants de substance entre les étapes de procédé précédemment mentionnées, dans le processus.

2. Procédé selon la revendication 1,
**caractérisé en ce qu**'
on décompose les vapeurs qui se forment dans la méthanolyse en une phase bouillant facilement et en une phase qui bout difficilement, on amène la fraction contenue dans les vapeurs d'ester triméthylique d'acide triméthylique (TMT) au produit de pot de la méthanolyse, on évacue par portions le produit de pot enrichi en TMT ou on l'ajoute à la fraction de résidu qui bout difficilement provenant du traitement du catalyseur.

3. Procédé selon la revendication 1 ou la revendication 2,
**caractérisé en ce qu**'
on ajoute à la fraction de résidu qui bout difficilement provenant de la distillation de l'ester brut avant l'entrée dans la méthanolyse, totalement ou partiellement, le résidu provenant de la distillation du DMP et on conduit le reste du résidu provenant de la distillation du DMP, à l'oxydation.

4. Procédé selon au moins l'une quelconque des revendications 1 à 3,
**caractérisé en ce qu**'
on conduit la fraction de résidu qui bout difficilement provenant de la distillation de l'ester brut par l'intermédiaire d'une méthanolyse, qui est mise en fonctionnement avec une introduction multiple de méthanol.

5. Procédé selon au moins l'une quelconque des revendications 1 à 4,
**caractérisé en ce qu**'
on fait passer la fraction de résidu qui bout difficilement provenant de la distillation de l'ester brut avant l'entrée dans la méthanolyse par une redistillation et/ou un stade préliminaire de méthanolyse.

6. Procédé selon la revendication 5,
**caractérisé en ce qu**'
on fait passer le distillat de la redistillation par une distillation de TMT et/ou on recycle partiellement dans la distillation de l'ester brut.

7. Procédé selon au moins l'une quelconque des revendications 1 à 6,
**caractérisé en ce que**
la quantité des résidus à haut point d'ébullition recyclés provenant de la récupération du catalyseur s'élève à > 0 à 20 % en poids de la quantité de DMT brut produite dans le processus en cours.
